# EUROPEAN PATENT SPECIFICATION

(11) **EP 2 640 382 B1**
(45) Date of publication and mention of the grant of the patent: **19.10.2016**
(21) Application number: 11785262.4
(22) Date of filing: 10.11.2011
(51) Int. Cl.: A61K 31/4174, A61P 27/06

(54) **PHARMACEUTICAL COMPOSITIONS COMPRISING (3-(1-(1H-IMIDAZOL-4-YL)ETHYL)-2-METHYLPHENYL)METHANOL**
PHARMAZEUTISCHE ZUSAMMENSETZUNGEN MIT (3-(1-(1H-IMIDAZOL-4-YL)ETHYL)-2-METHYLPHENYL)METHANOL
COMPOSITIONS PHARMACEUTIQUES COMPRENANT DU [3-(1-(1H-IMIDAZOL-4-YL)ÉTHYL)-2-MÉTHYLPHÉNYL]MÉTHANOL

(30) Priority: 16.11.2010 US 414180 P
(43) Date of publication of application: 25.09.2013
(60) Divisional application: 16176846.0
(73) Proprietor: ALLERGAN, INC., Irvine, CA 92612 (US)
(72) Inventor: DIBAS, Mohammed I., Laguna Niguel, California 92677 (US); DONELLO, John E., Dana Point, California 92629 (US); GIL, Daniel W., Corona Del Mar, California 92625 (US)
(74) Representative: Hoffmann Eitle
(86) International application number: PCT/US2011/060236
(87) International publication number: WO 2012/067941

(56) References cited:
- WO-A1-02/089804
- WO-A1-2010/093930

## Description

### BACKGROUND OF THE INVENTION

### 1. Field of the Invention

The present invention relates to a pharmaceutical composition containing (S) (3-(1-(1 H-imidazol-4-yl)ethyl)-2-methylphenyl)methanol or a pharmaceutically acceptable salt thereof for use in a method of treating elevated intraocular pressure or glaucoma.

### 2. Summary of the Related Art

[3-(1-(1H-imidazol-4-yl)ethyl)-2-methylphenyl]methanol is known as a selective modulator of the alpha-2 adrenergic receptors. Three alpha-1 and three alpha-2 adrenergic receptors have been characterized by molecular and pharmacological methods. Activation of these alpha receptors evokes physiological responses with useful therapeutic actions.

4-[1-(2,3-dimethylphenyl)ethyl]-3*H*-imidazole, generically known as medetomidine, is an alpha-2 adrenergic agonist, for use in the sedation of animals. The hydrochloride salt of the (S) enantiomer of medetomidine, generically known as dexmedetomidine, (S) 4-[1-(2,3-dimethylphenyl)ethyl]-3*H*-imidazole, is also indicated for use as a sedative or analgesic in cats and dogs.

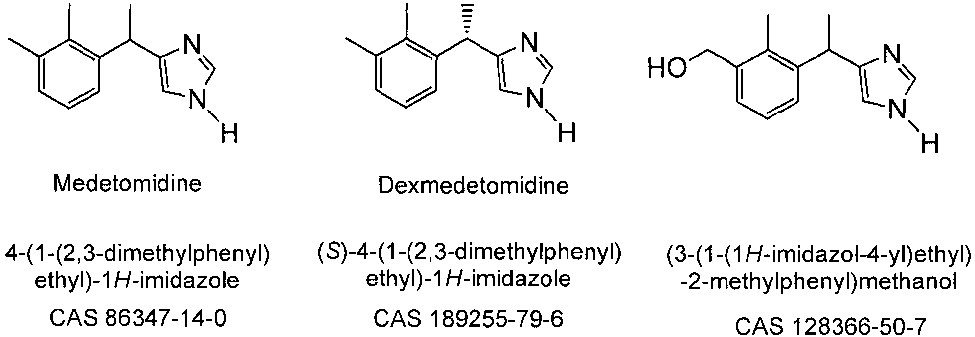

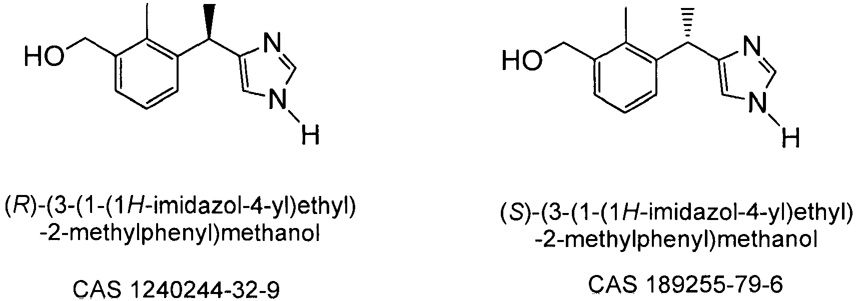

The metabolite of dexmedetomidine is (S) [3-(1-(1*H*-imidazol-4-yl)ethyl)-2-methylphenyl]methanol. Journal of Chromatography, (1997), 762, 281-291 by Hui, Y.-H et al. describes (S) [3-(1-(1*H*-imidazol-4-yl)ethyl)-2-methylphenyl]methanol together with its racemic mixture.

[3-(1-(1*H*-imidazol-4-yl)ethyl)-2-methylphenyl]methanol is described in "Synthesis of detomidine and medetomidine metabolites: 1,2,3-trisubstituted arenes with 4'(5')-imidazolylmethyl groups" in Journal of Heterocyclic Chemistry, (1993), 30(6), (1645-1651) by Stoilov et al.

Kavanagh, et al. describe [3-(1-(1H-imidazol-4-yl)ethyl)-2-methylphenyl]methanol in "Synthesis of Possible Metabolites of Medetomidine {1-(2,3-dimethylphenyl)-1-[imidazol-4(5)-yl]ethane" in Journal of Chemical Research, Synopses (1993), (4), 152-3.

[3-(1-(1*H*-imidazol-4-yl)ethyl)-2-methylphenyl]methanol is described by Salonen, et al. in "Biotransformation of Medetomidine in the Rat" in Xenobiotica (1990), 20(5), 471-80.

PCT publication WO 2010/093930 A1 discloses [3-(1-(1*H*-imidazol-4-yl)ethyl)-2-methylphenyl]methanol and its (S) and (R) enantiomers. These compounds are used to treat pain.

WO 02/089804 A1 discloses ophthalmic compositions containing an alpha-2 adrenergic agonist and a fatty acid component. Examples of the agonist include brimonidine.

### BRIEF DESCRIPTION OF THE DRAWING

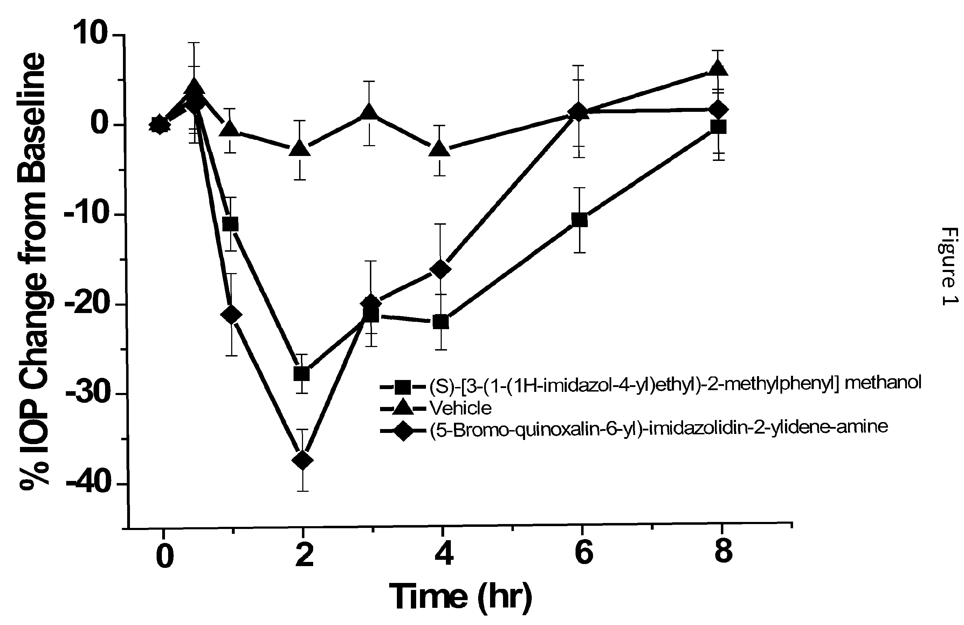

**Figure 1** shows that (S)-[3-(1-(1H-imidazol-4-yl)ethyl)-2-methylphenyl]methanol (**Compound 1**) has comparable efficacy to brimonidine (Alphagan P®) and provides a longer duration of intraocular pressure reduction than brimonidine.

### SUMMARY OF THE INVENTION

The adrenergic alpha-2 agonists play a key role in modulating aqueous humor formation and facilitating aqueous outflow; as a result these compounds lower intraocular pressure (IOP) in glaucomatous patients. Two drugs are currently prescribed for glaucoma patients: Apraclonidine (Lopidine®) and Brimonidine (Alphagan P®, available from Allergan, Inc.). While these drugs are effective at lowering elevated intraocular pressure, Alphagan P® is the only alpha-2 adrenergic approved only for a 3 times per day dosing regime, while Lopidine® is only approved for short term IOP control. Considering the aged glaucoma patient population, a 3 times per day dosing frequency is far from optimal and may result in poor patient compliance.

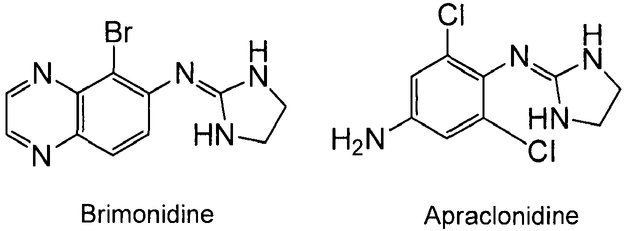

The present invention provides a pharmaceutical composition comprising (S) (3-(1-(1 H-imidazol-4-yl)ethyl)-2-methylphenyl)methanol or a salt thereof for use in a method of treating elevated intraocular pressure or glaucoma, wherein the method comprises topical administration of the composition to an affected eye of the patient once per day.

In an embodiment, the pharmaceutical composition comprises, consists essentially of or consists of a therapeutically effective amount of (S) [3-(1-(1H-imidazol-4-yl)ethyl)-2-methylphenyl]methanol, and the method comprises, consists essentially of or consists of administering the pharmaceutical composition to the affected eye of the patient, as a single dose, wherein the affected eye maintains an intraocular pressure less than the baseline intraocular pressure for at least eight (8) hours, preferably at least ten (10) hours, and more preferably at least twelve (12) hours, from the time of administration.

The term "baseline", as used herein, refers to the intraocular pressure measurement taken for the untreated eye.

The term "subject", as used herein, refers to a human patient.

The affected eye preferably maintains an intraocular pressure less than the baseline intraocular pressure throughout the day.

The composition that is used, as a single dose, to lower intraocular pressure for at least eight (8) hours, preferably at least ten (10) hours and more preferably for at least twelve (12) hours, may comprise from 0.01 to 5 percent by weight, preferably from 0.01 to 2 percent by weight, more preferably from 0.05 to 1 percent by weight, (S)-[3-(1-(1H-imidazol-4-yl)ethyl)-2-methylphenyl] methanol in a pharmaceutically-acceptable vehicle. Said composition is preferably formulated as an eye drop for topical administration.

The pharmaceutical composition is preferably formulated as a solution in water at a pH of 5.5 to 8.0, e.g. 6.9. While the precise regime is left to the discretion of the clinician, it is recommended that the solution be topically applied by placing one drop in each eye once a day.

Other ingredients which may be desirable to include in the composition include preservatives, co-solvents and viscosity building agents; sodium chloride, potassium chloride, calcium chloride dihydrate, magnesium chloride hexahydrate, boric acid and sodium borate decahydrate (as buffering agents); and purified water *(*Clinical Ocular Pharmacology by Jimmy D. Bartlett & Siret D. Jaanus, 2008, p 266).

Preservatives are required to prevent microbial contamination during use. Suitable preservatives include stabilized oxychloro complex (sold under the trademark Purite™), stabilized chlorine dioxide, benzalkonium chloride, thimerosal, chlorobutanol, methyl paraben, propyl paraben, phenylethyl alcohol, edetate disodium, sorbic acid, Onamer M, or other agents known to those skilled in the art *(*Review of Ophthalmology, June 2001, Robert Noecker, MD). A common side-effect of these preservatives is burning.

The present invention provides the improvement of exposing the patient to less preservative, since the (S)-[3-(1-(1H-imidazol-4-yl)ethyl)-2-methylphenyl]methanol containing composition is administered only once per a day, unlike the prior art alpha-2 adrenergic agonists which require three doses, daily, to control elevated intraocular pressure. Typically, the effective concentration of the preservative will range from 0.001% to 1% by weight, preferably from 0.01% to 0.5% by weight. In particular, the amount of stabilized oxychloro complex (Purite^{®}) can range from 0.001 to 0.01% by weight.

The solubility of the components of the composition may be enhanced by a surfactant or another appropriate co-solvent. Such co-solvents include polysorbate 20, 60, and 80, Pluronic^{®} F-68, F-84 and P-103, cyclodextrin, Solutol, and other agents known to those skilled in the art. Typically such co-solvents are employed at a level of from 0.01 % to 2% by weight.

Increase of the viscosity of an aqueous solution may be desirable to increase ocular absorption of the active compound, to decrease variability in dispensing the formulation, to decrease physical separation of components of a suspension or emulsion of the formulation and/or to otherwise improve the ophthalmic formulation. Suitable viscosity building agents include polyvinyl alcohol, polyvinyl pyrrolidone, methyl cellulose, hydroxypropyl methylcellulose, hydroxyethyl cellulose, carboxymethyl cellulose, hydroxypropyl cellulose, and other agents known to those skilled in the art. Such agents are typically employed at a level of from 0.01% to 2% by weight.

The following formulations are representative ophthalmic compositions of the invention for topical use for treating elevated intraocular pressure associated with glaucoma. In one example, the free base of (S)-[3-(1-(1H-imidazol-4-yl)ethyl)-2-methylphenyl]methanol is dissolved in sterile distilled water, hydrochloric acid is added and the hydrochloric salt of the compound is formed *in situ.* The solution is titrated with sodium hydroxide until the pH of the solution reaches about 8.0. The final concentration of (S)-[3-(1-(1H-imidazol-4-yl)ethyl)-2-methylphenyl]methanol is about 1% by weight. In another example, the free base of (S)-[3-(1-(1 H-imidazol-4-yl)ethyl)-2-methylphenyl]methanol is dissolved in sterile distilled water with boric acid, benzalkonium chloride and glycerin.

"Pharmaceutical composition," as used herein, means a composition that is suitable for administering to human patients for the treatment of disease. In a further embodiment, therefore, the compound of the invention is formulated as a pharmaceutically acceptable salt and is used together with one or more pharmaceutically acceptable excipients.

"Pharmaceutically acceptable salt" refers to those salts which retain the biological effectiveness and properties of the free base and which are obtained by reaction with inorganic acids such as hydrochloric acid, hydrobromic acid, sulfuric acid, nitric acid, phosphoric acid, methanesulfonic acid, ethanesulfonic acid, p-toluenesulfonic acid, salicylic acid and the like. The acid addition salt form of (S)-[3-(1-(1 H-imidazol-4-yl)ethyl)-2-methylphenyl]methanol that occurs in its free form as a base, can be obtained by treating the free base with an appropriate acid such as an inorganic acid, for example hydrochloric acid, hydrobromic acid, sulfuric acid, phosphoric acid, nitric acid and the like; or an organic acid such as acetic acid, hydroxyacetic acid, propanoic acid, lactic acid, pyruvic acid, malonic acid, fumaric acid, maleic acid, oxalic acid, tartaric acid, succinic acid, malic acid, ascorbic acid, benzoic acid, tannic acid, pamoic acid, citric acid, methylsulfonic acid, ethanesulfonic acid, benzenesulfonic acid, formic acid and the like (Handbook of Pharmaceutical Salts, P. Heinrich Stahal & Camille G. Wermuth (Eds), Verlag Helvetica Chemica Acta-Zürich, 2002, 329-345).

The compounds can also be administered as pharmaceutically acceptable quaternary salts known by those skilled in the art, which specifically include the quaternary ammonium salt of the formula -NY⁺Z⁻, wherein Y is hydrogen, alkyl, or benzyl, and Z is a counterion, including but not limited to, chloride, bromide, iodide, - O-alkyl, toluenesulfonate, methylsulfonate, sulfonate, phosphate, or carboxylate (such as fumarate, benzoate, succinate, acetate, glycolate, maleate, malate, fumarate, citrate, tartrate, ascorbate, benzoate, cinnamoate, mandeloate, benzyloate, and diphenylacetate).

The pharmaceutical composition may include a pharmaceutically acceptable carrier, diluent or excipient. The phrase "pharmaceutically acceptable" means that the carrier, diluent or excipient must be compatible with the other ingredients of the formulation and not deleterious to the recipient thereof.

The pharmaceutical composition can be used in the form of a solid, a solution, an emulsion, a dispersion, a patch, a micelle, a liposome, or the like, wherein the resulting composition contains one or more compounds of the present invention, as an active ingredient, in admixture with an organic or inorganic carrier or excipient. Invention compounds may be combined, for example, with the usual non-toxic, pharmaceutically acceptable carriers for solutions, emulsions, suspensions, and any other form suitable for use. The carriers which can be used include but are not limited to, glucose, lactose, gum acacia, gelatin, mannitol, starch paste, magnesium trisilicate, talc, corn starch, keratin, colloidal silica, potato starch, urea, medium chain length triglycerides, dextrans, and other carriers suitable for use in manufacturing preparations in solid, semisolid, or liquid form. In addition auxiliary, stabilizing, thickening and coloring agents and perfumes may be used. Invention compounds are included in the pharmaceutical composition in an amount sufficient to produce the desired effect upon the process or disease condition.

Since individual subjects may present a wide variation in severity of symptoms and each drug has its unique therapeutic characteristics, the precise mode of administration and dosage employed for each subject is left to the discretion of the practitioner. The actual amount of the compound to be administered in any given case will be determined by a physician taking into account the relevant circumstances, such as the severity of the condition, the age and weight of the patient, the patient's general physical condition, the cause of the condition, and the route of administration. Additionally, the formulations may be designed to delay release of the active compound over a given period of time, or to carefully control the amount of drug released at a given time during the course of therapy.

(S)-[3-(1-(1H-imidazol-4-yl)ethyl)-2-methylphenyl]methanol and its pharmaceutically-acceptable salts have extended alpha-2 adrenergic receptor agonist activity in lowering intraocular pressure and may be administered through different routes, including but not limited to topical eye drops, and in formulations that enhance the long duration of action such as a suspension, gel, or suitable drug delivery system (DDS) known in the art.

The present invention is not to be limited in scope by the exemplified embodiments, which are only intended as illustrations of specific aspects of the invention. Various modifications of the invention, in addition to those disclosed herein, will be apparent to those skilled in the art by a careful reading of the specification, including the claims, as originally filed. It is intended that all such modifications will fall within the scope of the appended claims.

The following assays and animal models are used to demonstrate the potency and selectivity of the compounds according to the invention.

### Example 1

### In-Vivo IOP Compound Screening

The experimental animals used were normotensive male Dutch-Belted rabbits (Myrtle's Rabbitry) over 6 months in age (n = 4/compound/dose screened). A single drop (50 µl) of the drug formulation was administered topically by pipette onto the right eye (treated eye) at approximately 0700 hours. IOP of the rabbits (treated and untreated eyes) was measured 0 hours before and at 0.5, 1, 2, 3, 4, 6 and 8 hours after topical eyedrop administration. IOP at the time of eyedrop administration (0 hours) was used as a baseline value. Prior to the tonometric measurements, 0.05% proparacaine (50 µl) was administered to each eye. Tonometric IOP measurements were obtained with a Mentor Pneumontonmeter. Additionally, all studies were masked. At least 1 week of wash-out time was allowed for each rabbit between dosings. All animals were examined for sedation, ocular irritation, and changes in pupil diameter throughout the course of the experiments.

(S)-[3-(1-(1H-imidazol-4-yl)ethyl)-2-methylphenyl]methanol (Compound 1) at 0.15% concentration lowered IOP for at least 6 hours in the treated eye of DB Rabbits, whereas the effect of brimonidine (0.15%) was minimal at 6 hrs. (S)-[3-(1-(1 H-imidazol-4-yl)ethyl)-2-methylphenyl]methanol showed very little effect in the contralateral eye whereas brimonidine showed a strong contralateral effect. The data is shown in **Figure 1****.**

### Example 2

### FLIPR Ca⁺² Influx Assay

HEK 293 cells stably expressing the bovine alpha-1A receptor, human alpha-2A receptor and the chimeric G protein G_{qi5}, were plated in poly-D-lysine coated 384-well plates at 20,000 - 40,000 cells per well and grown overnight in DMEM supplemented with 10% fetal bovine serum. For FLIPR (fluorometric image plate reader) evaluation, cells were washed twice with HBSS/HEPES buffer (1X Hanks Buffered Salt Solution, 20 mM HEPES, pH 7.4) prior to the addition of Fluo-4-AM (4 uM Fluo-4-AM, 0.04% pluronic acid in HBSS/HEPES buffer), a calcium-sensitive dye. Cells were loaded with dye for 40 minutes at 37°C, then washed 4 times with HBSS/HEPES buffer. For both the agonist and antagonist assay, the test compounds were tested between 0.64 nM and 10,000 nM.

For an agonist assay, the reaction was initiated by the addition of the appropriate dilutions of compounds and the transient calcium signal captured. The peak height of the calcium curve was determined and utilized for calculation of EC₅₀ and efficacy using ActivityBase. Norepinephrine was the standard full agonist used for evaluating alpha-1 and alpha-2 receptor activity.

**Table 1**

| *In vitro* pharmacology of (S)-[3-(1-(1H-imidazol-4-yl)ethyl)-2-methylphenyl]methanol at adrenergic receptor subtypes. | | | |
|---|---|---|---|
| **Entry** | **Compound** | **FLIPR Assay** | |
| | | **a1A** | **a2A** |
| 1 | Brimonidine | 600-2400 (0.3) | 5 (0.95) |
| 2 | (S)-[3-(1-(1 H-imidazol-4-yl)ethyl)-2-methylphenyl] methanol (Compound 1) | 340-2400 (0.7) | 25 (0.9) |

| | | | |
|---|---|---|---|
| EC50 in nM (efficacy). | | | |

## Claims

1. A pharmaceutical composition comprising (S) (3-(1-(1 H-imidazol-4-yl)ethyl)-2-methylphenyl)methanol or a salt thereof for use in a method of treating elevated intraocular pressure or glaucoma, wherein the method comprises topical administration of the composition to an affected eye of the patient once per day.

2. A pharmaceutical composition for use according to Claim 1, wherein the method involves topical administration of the composition as a single dose and the affected eye has an intraocular pressure less than the baseline intraocular pressure for at least eight (8) hours after administration.

3. A pharmaceutical composition for use according to Claim 1, wherein the affected eye maintains an intraocular pressure less than the baseline intraocular pressure for at least ten (10) hours after administration of the composition.

4. A pharmaceutical composition for use according to Claim 1, wherein the affected eye maintains an intraocular pressure less than the baseline intraocular pressure for at least twelve (12) hours after administration of the composition.

5. A pharmaceutical composition for use according to Claim 1, wherein the composition comprises from about 0.01% to about 5% by weight of (S) (3-(1-(1 H-imidazol-4-yl)ethyl)-2-methylphenyl)methanol or a salt thereof.

6. A pharmaceutical composition for use according to Claim 1, wherein the composition comprises from about 0.01% to about 2% by weight of (S) (3-(1-(1 H-imidazol-4-yl)ethyl)-2-methylphenyl)methanol or a salt thereof.

7. A pharmaceutical composition for use according to Claim 1, wherein the composition comprises from about 0.05% to about 1% by weight of (S) (3-(1-(1H-imidazol-4-yl)ethyl)-2-methylphenyl)methanol or a salt thereof.

8. A pharmaceutical composition for use according to Claim 1, wherein the composition further comprises from about 0.001% to about 1% by weight of a preservative.

9. A pharmaceutical composition for use according to Claim 1, wherein the composition further comprises from about 0.01% to about 0.5% by weight of a preservative.

10. A pharmaceutical composition for use according to Claim 1, wherein the composition further comprises from about 0.001% to about 0.01% by weight of a preservative.

11. A pharmaceutical composition for use according to Claim 1, wherein the composition further comprises from about 0.01% to about 2% by weight of a co-solvent.

12. A pharmaceutical composition for use according to Claim 1, wherein the composition further comprises from about 0.01% to about 2% by weight of a viscosity building agent.

## Patentansprüche

1. Pharmazeutische Zusammensetzung, umfassend (S)(3-(1-(1H-Imidazol-4-yl)ethyl)-2-methylphenyl)methanol oder ein Salz davon, zur Verwendung in einem Verfahren zur Behandlung von erhöhtem Augeninnendruck oder Glaukom, wobei das Verfahren die topische Verabreichung der Zusammensetzung an das betroffene Auge des Patienten einmal täglich umfasst.

2. Pharmazeutische Zusammensetzung zur Verwendung gemäss Anspruch 1, wobei das Verfahren die topische Verabreichung der Zusammensetzung als Einzeldosis einbezieht und das betroffene Auge für zumindest acht (8) Stunden nach Verabreichung einen Augeninnendruck aufweist, der geringer als der Ausgangswert des Augeninnendrucks ist.

3. Pharmazeutische Zusammensetzung zur Verwendung gemäss Anspruch 1, wobei das betroffene Auge für zumindest zehn (10) Stunden nach Verabreichung der Zusammensetzung einen Augeninnendruck beibehält, der geringer als der Ausgangswert des Augeninnendrucks ist.

4. Pharmazeutische Zusammensetzung zur Verwendung gemäss Anspruch 1, wobei das betroffene Auge für zumindest zwölf (12) Stunden nach Verabreichung der Zusammensetzung einen Augeninnendruck beibehält, der geringer als der Ausgangswert des Augeninnendrucks ist.

5. Pharmazeutische Zusammensetzung zur Verwendung gemäss Anspruch 1, wobei die Zusammensetzung etwa 0,01 bis etwa 5 Gew.% (S)(3-(1-(1H-Imidazol-4-yl)ethyl)-2-methylphenyl)methanol oder ein Salz davon umfasst.

6. Pharmazeutische Zusammensetzung zur Verwendung gemäss Anspruch 1, wobei die Zusammensetzung etwa 0,01 bis etwa 2 Gew.% (S)(3-(1-(1H-Imidazol-4-yl)ethyl)-2-methylphenyl)methanol oder ein Salz davon umfasst.

7. Pharmazeutische Zusammensetzung zur Verwendung gemäss Anspruch 1, wobei die Zusammensetzung etwa 0,05 bis etwa 1 Gew.% (S)(3-(1-(1H-Imidazol-4-yl)ethyl)-2-methylphenyl)methanol oder ein Salz davon umfasst.

8. Pharmazeutische Zusammensetzung zur Verwendung gemäss Anspruch 1, wobei die Zusammensetzung ferner etwa 0,001 bis etwa 1 Gew.% eines Konservierungsmittels umfasst.

9. Pharmazeutische Zusammensetzung zur Verwendung gemäss Anspruch 1, wobei die Zusammensetzung ferner etwa 0,01 bis etwa 0.5 Gew.% eines Konservierungsmittels umfasst.

10. Pharmazeutische Zusammensetzung zur Verwendung gemäss Anspruch 1, wobei die Zusammensetzung ferner etwa 0,001 bis etwa 0,01 Gew.% eines Konservierungsmittels umfasst.

11. Pharmazeutische Zusammensetzung zur Verwendung gemäss Anspruch 1, wobei die Zusammensetzung ferner etwa 0,01 bis etwa 2 Gew.% eines Co-Lösungsmittels umfasst.

12. Pharmazeutische Zusammensetzung zur Verwendung gemäss Anspruch 1, wobei die Zusammensetzung ferner etwa 0,01 bis etwa 2 Gew.% eines Viskosität aufbauenden Mittels umfasst.

## Revendications

1. Composition pharmaceutique comprenant du (S) (3-(1-(1H-imidazol-4-yl)éthyl)-2-méthylphényl)méthanol ou un sel de celui-ci pour utilisation dans un procédé de traitement d'une pression intraoculaire élevée ou d'un glaucome, dans laquelle le procédé comprend l'administration topique de la composition à un oeil affecté du patient une fois par jour.

2. Composition pharmaceutique pour utilisation selon la revendication 1, dans laquelle le procédé implique l'administration topique de la composition sous la forme d'une dose unique, et l'oeil affecté a une pression intraoculaire inférieure à la pression intraoculaire de base pendant au moins huit (8) heures après administration.

3. Composition pharmaceutique pour utilisation selon la revendication 1, dans laquelle l'oeil affecté conserve une pression intraoculaire inférieure à la pression intraoculaire de base pendant au moins dix (10) heures après administration de la composition.

4. Composition pharmaceutique pour utilisation selon la revendication 1, dans laquelle l'oeil affecté conserve une pression intraoculaire inférieure à la pression intraoculaire de base pendant au moins douze (12) heures après administration de la composition.

5. Composition pharmaceutique pour utilisation selon la revendication 1, dans laquelle la composition comprend environ 0,01 % à environ 5 % en poids de (S) (3-(1-(1H-imidazol-4-yl)éthyl)-2-méthylphényl)méthanol ou d'un sel de celui-ci.

6. Composition pharmaceutique pour utilisation selon la revendication 1, dans laquelle la composition comprend environ 0,01 % à environ 2 % en poids de (S) (3-(1-(1H-imidazol-4-yl)éthyl)-2-méthylphényl)méthanol ou d'un sel de celui-ci.

7. Composition pharmaceutique pour utilisation selon la revendication 1, dans laquelle la composition comprend environ 0,05 % à environ 1 % en poids de (S) (3-(1-(1H-imidazol-4-yl)éthyl)-2-méthylphényl)méthanol ou d'un sel de celui-ci.

8. Composition pharmaceutique pour utilisation selon la revendication 1, dans laquelle la composition comprend en outre environ 0,001 % à environ 1 % en poids d'un conservateur.

9. Composition pharmaceutique pour utilisation selon la revendication 1, dans laquelle la composition comprend en outre environ 0,01 % à environ 0,5 % en poids d'un conservateur.

10. Composition pharmaceutique pour utilisation selon la revendication 1, dans laquelle la composition comprend en outre environ 0,001 % à environ 0,01 % en poids d'un conservateur.

11. Composition pharmaceutique pour utilisation selon la revendication 1, dans laquelle la composition comprend en outre environ 0,01 % à environ 2 % en poids d'un co-solvant.

12. Composition pharmaceutique pour utilisation selon la revendication 1, dans laquelle la composition comprend en outre environ 0,01 % à environ 2 % en poids d'un agent viscogène.
